(19) Europäisches Patentamt
European Patent Office
Office européen des brevets

(11) Veröffentlichungsnummer: **0 224 275**
**B1**

(12) **EUROPÄISCHE PATENTSCHRIFT**

(45) Veröffentlichungstag der Patentschrift: **01.08.90**

(21) Anmeldenummer: **86116566.0**

(22) Anmeldetag: **28.11.86**

(51) Int. Cl.$^5$: **C 07 C 405/00,**
A 61 K 31/557,
C 07 C 229/38, C 07 C 229/40,
C 07 D 309/12

# (54) Neue Carbacycline, Verfahren zu ihrer Herstellung und ihre Verwendung als Arzneimittel.

(30) Priorität: **29.11.85 DE 3542745**

(43) Veröffentlichungstag der Anmeldung:
**03.06.87 Patentblatt 87/23**

(45) Bekanntmachung des Hinweises auf die Patenterteilung:
**01.08.90 Patentblatt 90/31**

(84) Benannte Vertragsstaaten:
**AT BE CH DE ES FR GB GR IT LI LU NL SE**

(56) Entgegenhaltungen:
**EP-A-0 086 611**
**WO-A-86/00895**

(73) Patentinhaber: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Vorbrüggen, Helmut, Prof.**
**Wilkestrasse 7**
**D-1000 Berlin 27 (DE)**
Erfinder: **Klar, Ulrich, Dr.**
**Helmkrautstrasse 21**
**D-1000 Berlin 27 (DE)**
Erfinder: **Nieuweboer, Bob**
**Bärbelweg 16A**
**D-1000 Berlin 27 (DE)**
Erfinder: **Stürzebecher, Claus Steffen, Dr.**
**Brigittenstrasse 6a**
**D-1000 Berlin 27 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 224 275 B1

**Beschreibung**

Die Erfindung betrifft neue Carbacyclin-Derivate, Verfahren zu ihrer Herstellung sowie ihre Verwendung als Arzneimittel. In der US-Patentschrift 4,420,632 werden 9-alkylierte Carbacyclin-Derivate beschrieben, die antithrombotische, antisekretorisiche und bronchiodilatierende Eingschaften besitzen. Sie wirken außerdem thrombozytenaggregationshemmend. Aus der europäischen Patentanmeldung EP 86 611 sind Carbacyoline bekannt, die in 9-Position durch Halogenmethyl oder Hydroxymethyl substituiert sind und die als weitere Substituierten ungesättigte Alkylgruppen tragen, wobei die Doppel -und Dreifache bindungen in direkter Nachbarscahft zum Bicyclin angeordnet sind. Ihre pharmakologischen Eigenschaften werden als antiulcer und antiasthmatisch beschrieben.

Es wurde gefunden, daß sich in 9-Stellung kettenverlängerte Carbacyclin-Analoga mit einer reaktiven Gruppe in ω-Stellung bei nur geringem Verlust an biologischer Aktivität an polymere Träger binden lassen. Die erfindungssgemäßen Verbindungen sind zur Inhibierung der Thrombozytenaggregation, zur Blutdrucksenkung über eine Vasodilation, zur Hemmung der Magensäurèsektretion sowie nach chemischer Verknüpfung mit Proteinen zur Darstellung von Antikörpern zu Carbacyclinen geeignet.

Die Erfindung betrifft Carbacyclinderivate der allgemeinen Formel I

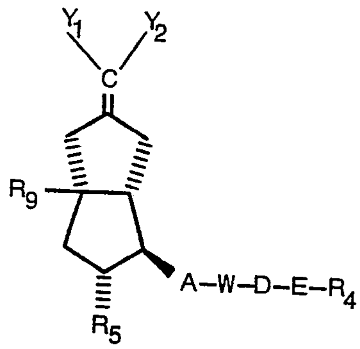

(I),

worin

$Y_1$ den Rest $—CH_2—X(CH_2)_n—R_1$ oder den Rest 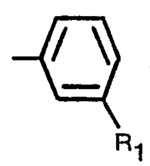 ,

n 1 oder 3,

$R_1$ den Rest 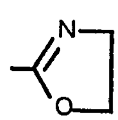 , den Rest 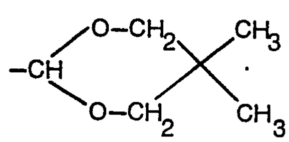 ,

$—COCH_3$, $COOR_2$, wobei $R_2$ Wasserstoff oder gegebenenfalls durch Halogen, Phenyl, $C_1—C_4$—Alkoxy oder $C_1—C_4$—Dialkyl-amino substituiertes Alkyl mit 1—10 C-Atomen, Cycloalkyl, Aryl oder einen heterocyclischen Rest bedeuten kann, oder den Rest $CONHR_3$ mit $R_3$ in der Bedeutung Wasserstoff oder eines Alkanoyl- oder Alkansulfonylrestes mit je 1—10 C-Atomen darstellt,

$R_9$ den Rest $—CH_2)_m—R_6$ oder den Rest

$$—(CH_2)_{m-o}—[Z_1—(CH_2)_{m-p}]_x—[Z_2—(CH_2)_{m-q}]_y—R_6,$$

m = 2—20

o, p und q $\leq 1_9$

x, y = 0, 1 oder 2,

$Z_1$ eine cis—CH=CH-Gruppe, eine trans—CH=CH—Gruppe oder eine —C≡C—Gruppe darstellt, wobei jede dieser Gruppen mindestens durch eine Methylengruppe vom C-9 Kohlenstoffatom des Carbacyclinbicyclus getrennt sein muß,

$Z_2$ Sauerstoff, Schwefel, eine NH— oder eine N-Methylgruppe bedeutet,

$R_6$ Amino, Methylamino, Hydroxy, Carboxy oder Merkapto bedeutet,

X eine Sauserstoffatom oder eine Methylengruppe,

$Y_2$ Wasserstoff oder Fluor,

A eine $—CH_2—CH_2—$, trans —CH=CH— oder —C≡C—Gruppe,

W eine freie oder funktionell abgewandelte Hydroxymethylengruppe oder eine freie oder funktionell abgewandelte

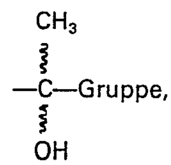

wobei die OH-Gruppe α- oder β-ständig sein kann,

D die Gruppe

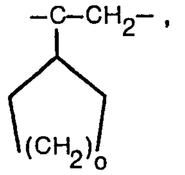

eine geradkettige, gesättigte Alkylengruppe mit 1—5 C-Atomen, eine verzweigte gesättigte oder eine geradkettige oder verzweigte ungesättigte Alkylengruppe mit 2—5 C-Atomen, die gegebenenfalls durch Fluoratome substituiert sein können,

o 1, 2 oder 3 bedeutet,

E eine Direktbindung, eine —C≡C—Gruppe oder eine $—CH=CR_7—$ Gruppe darstellt, wobei $R_7$ ein Wasserstoffatom, eine Alkylgruppe mit 1—5 C-Atomen oder Halogen bedeuten,

$R_4$ eine Alkylgruppe mit 1—10 C-Atomen, eine Cycloalkylgruppe mit 3—10 C-Atomen oder eine gegebenenfalls substituierte Arylgruppe mit 6—10 C-Atomen oder eine heterocyclische Gruppe,

$R_5$ eine freie oder funktionell abgewandelte Hydroxygruppe, und, falls $R_2$ die Bedeutung eines Wasserstoffatom hat, deren Salze mit physiologisch verträglichen Basen bedeuten.

Als Alkylgruppen $R_2$ sind gerad- oder verzweigtkettige Alkylgruppen mit 1—10 C-Atomen zu betrachten, wie beispielsweise Methyl, Äthyl, Propyl, Isopropyl, Butyl, Isobutyl, tert.-Butyl, Pentyl, Isopentyl, Neopentyl, Heptyl, Hexyl, Decyl.

Die Alkylgruppen $R_2$ können gegebenenfalls 1 bis mehrfach substituiert sein durch Halogenatome, $C_1$—$C_4$-Alkoxygruppen, Phenyl und Di-$C_1$—$C_4$-alkylamine. Bevorzugt sind solche Alkylgruppen, die einfach substituiert sind.

Als Substituenten seien beispielsweise genannt Fluor-, Chlor- oder Bromatome, Phenyl, Dimethylamino, Diäthylamino, Methoxy, Äthoxy.

Als bevorzugte Alkylgruppen $R_2$ sind solche mit 1—4 C-Atomen, wie z.B. Methyl, Äthyl, Propyl, Dimethylaminopropyl, Isobutyl, Butyl zu nennen.

Als Arylgruppen $R_2$ kommen sowohl substituierte wie auch unsubstituierte Arylgruppen in Betracht, wie beispielsweise Phenyl, 1-Naphthyl und 2-Naphthyl, die jeweils substituiert sein können durch 1—3 Halogenatome, eine Phenylgruppe, 1—3 Alkylgruppen mit jeweils 1—4 C-Atomen, eine Chlormethyl-, Fluormethyl-, Trifluormethyl-, Carboxyl-, Hydroxy- oder Alkoxygruppe mit 1—4 C-Atomen.

Bevorzugt sind die Substituenten in 3- und 4-Stellung am Phenylring, zum Beispiel durch Fluor, Chlor, Alkoxy oder Trifluormethyl oder in 4-Stellung durch Hydroxy.

Die Cycloalkylgruppe $R_2$ kann im Ring 4—10, vorzugsweise 5 und 6 Kohlenstoffatome enthalten. Die Ringe können durch Alkylgruppen mit 1—4 Kohlenstoffatomen substituiert sein. Beispielsweise seien genannt Cyclopentyl-, Cyclohexyl, Methylcyclohexyl und Adamantyl.

Als heterocyclische Gruppen $R_2$ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 3-Furyl, 2-Thienyl, 3-Thienyl, 2-Pyridyl, 3-Pyridyl, 4-Pyridyl u.a.

Als Säurerest $R_3$ kommen physiologisch verträgliche Säurereste in Frage. Bevorzugte Säuren sind organische Carbonsäuren und Sulfonsäuren mit 1—10 C-Atomen, die der aliphatischen, cycloaliphatischen, aromatischen, aromatischaliphatischen und heterocyclischen Reihe angehören. Diese Säuren können gesättigt, ungesättigt und/oder mehr-basisch und/oder in üblicher Weise substituiert sein. Als Beispiele für die Substituenten seien $C_1$—$C_4$-Alkyl, Hydroxy-, $C_1$—$C_4$-Alkoxy-, Oxo- oder Aminogruppen oder Halogenatome (F, Cl, Br) erwähnt.

Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diäthylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenoxyessigsäure, Methoxyessigsäure, Äthoxyessigsäure, Mono-, Di- und Trichloressigsäure, Aminoessigsäure, Diäthylaminoessigsäure, Piperidinoessigsäure, Morpholinoessigsäure, Milchsäure, Bernsteinsäure, Adipinsäure, Benzoesäure, mit Halogen-, Trifluormethyl-, Hydroxy-, Alkoxy- oder Carboxy-Gruppen substituierte Benzoesäuren, Nikotinsäure, Isonikotinsäure, Furan-2-carbonsäure, Cyclopentylpropionsäure, Als besonders bevorzugte Acylreste werden solche mit bis zu 4 Kohlenstoffatomen betrachtet. Als Sulfosäuren kommen beispielsweise Methansulfonsäure, Äthansulfonsäure, Isopropansulfonsäure, β-Chloräthansulfonsäure, Butansulfonsäure, Cyclopentansulfonsäure, Cyclohexansulfonsäure, Benzolsulfonsäure, p-Toluolsulfonsäure, p-Chlorbenzolsulfonsäure, N,N-Dimethylaminosulfonsäure, N,N-Diäthylaminosulfonsäure, N,N-Bis-(β-chloräthyl)-aminosulfonsäure, N,N-Diisobutylaminosulfonsäure, N,N-Dibutylaminosulfonsäure, Pyrrolidino-, Piperidino-, Piperazino-, N-Methylpiperazino- und Morpholinosulfonsäure in Frage, wobei Sulfonsäuren mit bis zu 4 C-Atomen besonders bevorzugt sind.

Die Hydroxygruppen $R_5$ und in W konnen funktionell abgewandelt sein, beispielsweise durch Verätherung oder Veresterung, wobei die freien oder abgewandelten Hydroxygruppen in W α- oder β-ständig sein können, wobei freie Hydroxygruppen bevorzugt sind.

Als Äther- und Acylreste kommen die dem Fachmann bekannten Reste in Betracht. Bevorzugt sind leicht abspaltbare Ätherreste wie beispielsweise der Tetrahydroxpyranyl-, Tetrahydrofuranyl, α-Äthoxyäthyl-, Trimethylsilyl-, Dimethyl-tert.-butyl-silyl- und Tri-p-benzyl-silylrest. Als Acylreste kommen die gleichen wie für $R_3$ genannt in Frage; namentlich genannt seien beispielswise Actyl, Propionyl, Butyryl, Benzoyl.

Als Alkylgruppe $R_4$ kommen gerad- und verzweigtkettige, gesättigte und ungesättigte Alkylreste, vorzugsweise gesättigte, mit 1—10, insbesondere 1—4 C-Atomen, in Frage.

Beispielsweise genannt seien Methyl-, Äthyl-, Propyl-, Butyl-, Isobutyl-, tert.-Butyl-, Pentyl-, Hexyl-, Heptyl-, Octyl-, Butenyl-, losbutenyl-, Propenyl-, Pentenyl-, Hexenyl-.

Die Cycloalkylgruppe $R_4$ kann im Ring 3—10, vorzugsweise 5 und 6 Kohlenstoffatome enthalten. Die Ringe können durch Alkylgruppen mit 1—4 Kohlenstoffatomen substituiert sein. Beispielsweise seien genannt Cyclopentyl-, Cyclohexyl, Methyl-cyclohexyl und Adamantyl.

Als substituierte bzw. unsubstituierte Arylgruppen $R_4$ kommen beispielsweise in Betracht: Phenyl, 1-Naphthyl und 2-Naphthyl, die jeweils substituiert sein können durch 1—3 Halogenatome, eine Phenylgruppe, 1—3 Alkylgruppen mit jeweils 1—4 C-Atomen, eine Chlormethyl-, Fluormethyl-, Trifluormethyl-, Carboxyl-, $C_1$—$C_4$-Alkoxy- oder Hydroxygruppe. Bevorzugt ist die Substitution in 3- und 4-Stellung am Phenylring zum Beispiel durch Fluor, Chlor, $C_1$—$C_4$-Alkoxy oder Trifluormethyl oder in 4-Stellung durch Hydroxy.

Als heterocyclische Gruppen $R_4$ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, 3-Furyl, 3-Thienyl u.a.

Als Alkylengruppe D kommen geradkettige oder verzweigtkettige, gesättigte und ungesättigte Alkylenreste, vorzugsweise gesättigte mit bis zu 5 C-Atomen, in Frage, die gegebenenfalls durch Fluoratome, 1,2-Methylen, 1,1-Trimethylen, 1,1-Tetramethylen oder 1,1-Pentamethylen substituiert sein können. Beispielsweise seien genannt: Methylen, Fluormethylen, Äthylen, 1,2-Propylen, Äthyläthylen, Trimethylen, Tetramethylen, Pentamethylen, 1-Methyltetramethylen, 1-Methyl-trimethylen, 1,1-Trimethylenäthylen, 1,1-Tetramethylenäthylen.

Besonders bevorzugte Verbindungen dieser Erfindung sind solche mit E als —C—C≡C— oder —C=$CR_7$—, worin $R_7$ eine Alkylgruppe mit 1—5 C-Atomen darstellt.

Als Alkylgruppe $R_7$ mit 1—5 C-Atomen kommen die für die Alkylgruppe $R_4$ bereits genannten Gruppe in Frage.

Mit $R_7$ als Halogen sind Fluor, Chlor und Brom gemeint.

Für $R_9$ als $-(CH_2)_m-R_6$ kommen Alkylengruppen mit 2—20 C-Atomen in Betracht, die noch eine oder mehrere Gruppen $Z_1$ oder $Z_2$ enthalten können wie $-(CH_2)_{m-o}-[Z_1-(CH_2)_{m-p}]_x-[Z_2-CH_2)_{m-q}]_y-R_6$, wobei m = 2—20 und o, p und q zusammen −16 ist, wie z.B.

$$-(CH_2)_5-NH_2,\ -(CH_2)_6-NHCH_3,\ -(CH_2)_2-O-(CH_2)_2-COOH,\ -(CH_2)_2-O-(CH_2)_3-NHC_2,$$

$$-(CH_2)_3-O-(CH_2)_3-NH_2,\ -(CH_2)_2-O-(CH_2)_2-O-(CH_2)_2OH,$$

$$-(CH_2)_3-\underset{\displaystyle CH_3}{\underset{|}{N}}-(CH_2)_3-NH_2,$$

$$-CH_2-C\equiv C-(CH_2)_2-NH_2,\ -(CH_2)_2-C\equiv C-(CH_2)_2-NH_2,\ -(CH_2)_2-C\equiv C-(CH_2)_2-O-(CH_2)_2-SH \text{ usw.}$$

Zur Salzbildung mit den freien Säuren ($R_2$ = H) sind anorganische und organische Basen geeignet, wie sie dem Fachmann zur Bildung physiologisch verträglicher Salze bekannt sind. Beispielsweise seien genannt: Alkalihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methylglucamin, Morpholin, Tris-(hydroxymethyl)-methylamin usw.

Die Erfindung betrifft ferner Verfahren zur Herstellung der erfindungsgemäßen Carbacycline der allgemeinen Formel I, die dadurch gekennzeichnet sind, daß man a) eine Verbindung der allgemeinen Formel IV

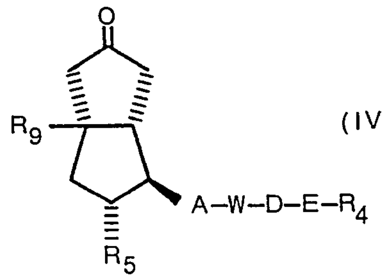

(IV),

worin A, W, D, E, $R_4$, $R_5$ und $R_9$ die bereits angegebenen Bedeutungen haben, und mit einem Wittigreagenz der allgemeinen Formeln V und VI bzw. einem Dianion der Formel VII

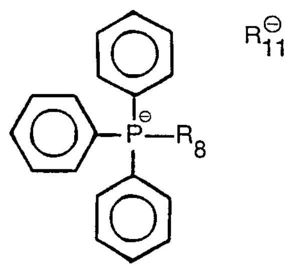

(V),

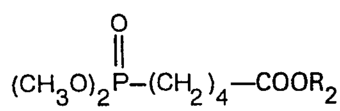

(VI)

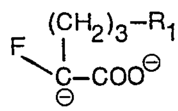

(VII)

worin

$R_2$ die genannte Bedeutung und $R_8$ die Reste $-CH_2-CH_2-X-$

$(CH_2)_n-R_1$ oder 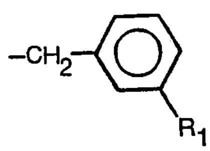

mit den bereits erwähnten Bedeutungen für X, n und $R_1$ und $R_{11}$ Brom oder Chlor bedeuten, in Gegenwart von K-tert.-Butylat umsetzt oder b) eine Verbindung der allgemeinen Formel VIII, die man aus dem entsprechenden 4-Ester durch DIBAH-Reduktion erhält,

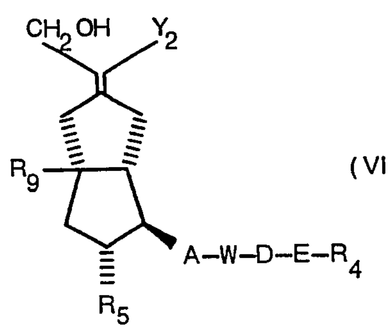

(VIII),

worin A, W, D, E, $R_4$, $R_5$ $R_9$ und $Y_2$ die bereits angegebenen Bedeutungen haben, gegebenenfalls nach Schutz anwesender freier Hydroxy- oder Aminogruppen mit einem Halogenalkansäurederivat der allgemeinen Formel IX,

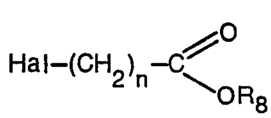

(IX),

wobei n 1 oder 3, Hal ein Chlor- oder Bromatom und $R_8$ einen Alkylrest mit 1—4 C-Atomen oder ein Alkalimetall bedeuten, in Gegenwart einer Base veräthert und gegebenenfalls anschließend in beliebiger

Reihenfolge Isomere trennt und/oder geschützte Hydroxygruppen freisetzt und/oder freie Hydroxygruppen verestert, veräthert und/oder eine freie Carboxylgruppe verestert und/oder eine veresterte Carboxylgruppe verseift oder eine Carboxylgruppe in ein Amid oder mit einer physiologisch verträglichen Base in ein Salz überführt.

Die Umsetzung der Verbindung der allgemeinen Formel VIII mit einem Halogenalkansäurederivat der allgemeinen Formel IX wird bei Temperaturen von 0°C bis 100°C, vorzugsweise 10° bis 80°C, in einem aprotischen Lösungsmittel oder Lösungsmittelgemisch, beispielsweise Dimethylsulfoxid, Dimethylformamid, Tetrahydrofuran usw., vorgenommen. Als Basen kommen die dem Fachmann für Verätherungen bekannten Basen in Frage, beispielsweise Natriumhydrid, Kalium-tert.-butylat, Butyllithium, usw.

Die Ausgangsverbindungen der Formel VI erhält man durch Umsetzung von Verbindungen der Formel II

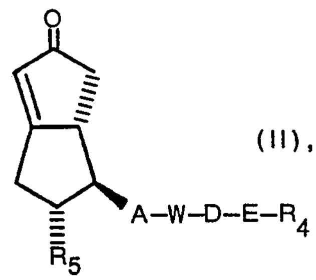

(II),

mit Grignard-Reagenzien der Formel III

$$R_{10}—[(CH_2)_{m-q}—Z_2]_y—[(CH_2)_{m-p}—Z_1]_x—(CH_2)_{m-o}—\text{Mg-Halogen} \quad \text{(III)},$$

worin $R_4$, $R_5$, A, W, D, E, $Z_1$, $Z_2$ m, o, p, q die angegebenen Bedeutungen haben, in Gegenwart von Kupfer (II) salzen und durch anschließende Einführung der oberen Seitenkette durch Wittig-Reaktion der 5-Ringcarbonylgruppe und Umwandlung der Gruppe $R_{10}$ in die Gruppe $R_6$. Für die Gruppe $R_{10}$ kommen geschützte Aminogruppen wie z.B. durch 1,1,4,4-Tetramethyl-1,4-dichlordisilyläthan oder durch Phthalsäureanhydrid bzw. durch andere typische Aminoschutzgruppen geschützte Aminogruppen sowie durch tert.-Butyldiphenylsilyl- oder THP-Gruppen geschützte Hydroxylgruppen sowie durch Umwandlung in Orthoester oder Oxazoline-geschützte Carboxylgruppen in Betracht.

Nach Abspaltung der Schutzgruppen vom Stickstoff bzw. nach chemischer Umwandlung der Hydroxy- oder Amidgruppen in Aminogruppen erhält man die gewünschten Substituenten $R_6$=$NH_2$. Für diese Umwandlung einer Hydroxygruppe in eine Aminogruppe kann man die Mitsunobu-Reaktion (vergl. Synthesis 1, 1981) bzw. die Reduktion des Azids zum Amin verwanden.

Die Verseifung der Carbacyclinester wird nach den dem Fachmann bekannten Methoden durchgeführt, beispielsweise mit basischen Katalysatoren.

Die Einführung der Estergruppe $COOR_2$ für $R_1$, bei welcher $R_2$ eine Alkylgruppe mit 1—10 C-Atomen darstellt, erfolgt nach den dem Fachmann bekannten Methoden. Die Carboxyverbindungen werden beispielsweise mit Diazokohlenwasserstoffen in an sich bekannter Weise umgesetzt. Die Veresterung mit Diazokohlenwasserstoffen erfolgt zum Beispiel dadurch, daß man eine Lösung des Diazokohlenwasserstoffes in einem inerten Lösungsmittel, vorzugsweise in Diäthyläther mit der Carboxyverbindung in dem gleichen oder in einem anderen inerten Lösungsmittel, wie zum Beispiel Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. *8,* Seiten 389—394 (1954)].

Die Einführung des Estergruppe $COOR_2$ für $R_1$, bei welcher $R_2$ eine substituierte oder unsubstituierte Arylgruppe darstellt, erfolgt nach den dem Fachmann bekannten Methoden. Beispielsweise werden die Carboxyverbindungen mit den entsprechenden Arylhydroxyverbindungen mit Dicyclohexylcarbodiimid in Gegenwart einer geeigneten Base, beispielsweise Pyridin oder Triäthylamin, in einem inerten Lösungsmittel umgesetzt. Als Lösungsmittel kommen Methylenchlorid, Äthylenchlorid, Chloroform, Essigester, Tetrahydrofuran, vorzugsweise Chloroform, in Frage, Die Reaktion wird bei Temperaturen zwischen −30°C und +50°C, vorzugsweise bei +10°C, durchgeführt.

Die Carbacyclin-Derivate der allgemeinen Formel I mit $R_1$ in der Bedeutung einer Carboxygruppe können mit geeigneten Mengen der entsprechenden anorganischen Basen unter Neutralisierung in Salze überführt werden. Beispielsweise erhält man beim Lösen der entsprechenden PG-Säuren in Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste angorganische Salz.

Die Herstellung der Aminsalze erfolgt in üblicher Weise. Dazu wird die PG-Säure zum Beispiel in einem geeigneten Lösungsmittel, wie Äthanol, Aceton, Diäthyläther oder Benzol gelöst und mindestens die

stöchiometrische Menge des Amins dieser Lösung zugesetzt. Dabei fällt das Salz gewöhnlich in fester Form an oder wird nach Verdampfen des Lösungsmittels in üblicher Weise isoliert.

Die funktikonelle Abwandlung der freien OH-Gruppen erfolgt nach den dem Fachmann bekannten Methoden. Zur Einführung der Ätherschutzgruppen wird beispielsweise mit Dihydropyran in Methylenchlorid oder Chloroform unter Verwendung eines sauren Kondensationsmittels, wie zum Beispiel p-Toluolsulfonsäure, umgesetzt. Das Dihydropyran wird im Überschuß angewandt, vorzugsweise in der 4- bis 10-fachen Menge des theoretischen Bedarfs. Die Umsetzung ist normalerweise bei 0°C—30°C nach 15—30 Minuten beendet.

Die Einführung der Acylschutzgruppen erfolgt, indem man eine Verbindung der allgemeinen Formel I in an sich bekannter Weise mit einem Carbonsäurederivat, wie zum Beispiel Säurechlorid, Säureanhydrid u.a., umsetzt.

Die Freisetzung einer funktionell abgewandelten OH-Gruppe zu den Verbindungen der allgemeinen Formel I erfolgt nach bekannten Methoden. Beispielsweise wird die Abspaltung von Ätherschutzgruppen in einer wäßrigen Lösung einer organischen Säure, wie zum Beispiel Essigsäure, Propionsäure u.a. oder in einer wäßrigen Lösung einer anorganischen Säure, wie zum Beispiel Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20°C und 80°C durchgeführt.

Die Abspaltung der Silylätherschutzgruppen erfolgt beispielsweise mit Tetrabutylammoniumfluorid. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0°C und 80°C durchgeführt.

Die Verseifung der Acylgruppen erfolgt beispielsweise mit Alkali- oder Erdalkali-carbonaten oder -hydroxyden in einem Alkohol oder der wäßrigen Lösung eines Alkohols. Als Alkohole kommen aliphatische Alkohole in Betracht, wie zum Beispiel Methanol, Äthanol, Butanol usw. vorzugsweise Methanol. Als Alkalicarbonate und -hydroxyde seien Kalium- und Natriumsalze genannt, bevorzugt sind jedoch die Kaliumsalze. Als Erdalkalicarbonate, und -hydroxyde sind beispielsweise geeignet Calciumcarbonat, Calciumhydroxyd und Bariumcarbonat. Die Umsetzung erfolgt bei −10°C bis 70°C, vorzugsweise bei 25°C.

Die Einführung der Amidgruppe $CONHR_3$ für $R_1$ erfolgt nach den dem Fachmann bekannten Methoden. Die Carbonsäuren der allgemeinen Formel I ($R_2$=H) werden zunächst in Gegenwart eines tertiären Amins, wie beispielsweise Triäthylamin, mit Chlorameisensäureisobutylester in das gemischte Anhydrid überführt. Die Umsetzung des gemischten Anhydrids mit dem Alkalisalz des entsprechenden Amids oder mit Ammoniak ($R_3$=H) erfolgt in einem inerten Lösungsmittel oder Lösungsmittelgemisch, wie beispielsweise Tetrahydrofuran, Dimethoxyäthan, Dimethylformamid, Hexamethylphosphorsäuretriamid, bei Temperaturen zwischen −30°C und +60°C, vorzugsweise bei 0°C bis 30°C.

Eine weitere Möglichkeit für die Einführung der Amidgruppe $CONHR_3$ für $R_1$ besteht in der Umsetzung einer 1-Carbonsäure der allgemeinen Formel I ($R_2$=H), in der freie Hydroxygruppen intermediär geschützt sind, mit Verbindungen der allgemeinen Formel X

$$O = C = N - R_3 \qquad (X)$$

worin $R_3$ die obenangegebene Bedeutung hat.

Die Umsetzung der Verbindung der allgemeinen Formel I ($R_1$=COOH) mit einem Isocyanat der allgemeinen Formel VIII erfolgt gegebenenfalls unter Zusatz eines tertiären Amins, wie z.B. Triäthylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diäthyläther, Toluol, bei Temperaturen zwischen −80°C bis 100°C, vorzugsweise bei 0°C bis 30°C, vorgenommen werden.

Enthält das Ausgangsprodukt OH-Gruppen im Prostanrest, so werden diese OH-Gruppen auch zur Reaktion gebracht. Werden letztlich Endprodukte gewünscht, die freie Hydroxylgruppen im Prostanrest enthalten, geht man zweckmäßigerweise von Ausgangsprodukten aus, in denen diese durch vorzugsweise leicht abspaltbare Äther- oder Acylreste intermediär geschützt sind.

Alle übrigen Verbindungen der Formel I können nach Verfahren hergestellt werden, wie sie in den Offenlegungsschriften DE—OS 28 45 770, 3237 200, 33 22 893 und 34 05 181 beschrieben werden.

Die Carbacycline der Formel I, in denen $R_9$ den Rest $—(CH_2)_m—R_6$ bzw. $—(CH_2)_{m-o}—[Z_1—(CH_2)_{m-p}]_x—[Z_2—(CH_2)_{m-q}]_y—R_6$ mit $R_6$ als $NH_2$—, $NHCH_3$—, OH—, COOH—, oder SH-Gruppe bedeutet, lassen sich sehr gut ohne größeren Verlust an biologischer Aktivität an polymere Träger binden. Durch die neuen Carbacycline wird verhindert, daß sich an der Oberfläche dieser polymeren Träger wie z.B. Gefäßprothesen oder künstlichen Herzklappen Thrombozytenaggregate bilden. Nach chemischer Verknüpfung mit Proteinen eignen sich die Verbindungen der Formel I zur Darstellung von Antikörpern zu Prostacyclinen der allgemeinen Formel I.

Die Verbindungen dieser Erfindung eignen sich ferner zur Therapie von Ekrankungen des cardiovaskulären Systems, des Magens, des Pankreas, der Leber und der Niere. Sie wirken blutdrucksenkend und bronchodilatorisch. Sie sind weiterhin geeignet zur Hemmung der Thrombozyten-Aggregation. Folglich

stellen die neuen Carbacyclin-Derivate der Formel I wertvolle pharmazeutische Wirkstoffe dar. Darüber hinaus weisen sie bei ähnlichem Wirkungsspektrum, verglichen mit entsprechenden Prostaglandinen und Prostacyclinen, eine höhere Spezifität und vor allem eine wesentlich längere Wirksamkeit auf. Im Vergleich zu $PGI_2$ zeichnen sie sich durch größere Stabilität aus. Die hohe Gewebsspezifität der neuen Carbacycline zeigt sich bei der Untersuchung an glattmuskulären Organen, wie z.B. am Meerschweinchenileum oder an der isolierten Kaninchentrachea, wo eine wesentlich geringere Stimulation zu beobachten ist als bei der Applikation natürlicher Prostaglandine vom E-, A- oder F-Typ.

Die neuen Carbacyclin-Analoga besitzen die für Prostacycline typischen Eigenschaften, wie z.B. Senkung des peripheren arteriellen und koronaren vaskulären Widerstandes, Inhibierung der Thrombozytenaggregation und Auflösung von Plättchenthromben, myocardiale Zytoprotektion, Senkung des systemischen Blutdruckes ohne zugleich Schlagvolumen und koronare Durchblutung zu senken; Behandlung von Schlanganfall, Prophylaxe und Therapie koronarer Herzerkrankungen, koronarer Thrombose, des Herzinfarkts, peripherer Arterienerkrankungen, Arterioaklerose und Thrombose, Prophylaxe und Therapie ischaemischer Attacken des ZNS-Systems, Therapie des Schocks, Inhibierung der Bronchokonstriktion, Inhibierung der Magensäuresekretion und Zytoprotektion der Magen- und Darmschleimhaut; Zytoprotektion in der Leber, im Pankreas und in der Niere, antiallergische Eigenschaften, Senkung des pulmonalen vaskulären Widerstandes und des pulmonalen Blutdruckes, Förderung der Nierendurchblutung, Anwendung anstelle von Heparin oder als Adjuvans bei der Dialyse oder Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung, Behandlung von Asthma, etc. Außerdem besitzen die neuen Carbacyclinanaloga antiproliferative Eigenschaften. Die neuen Carbacycline können außerdem in Kombination z.B. mit β-Blockern oder Diuretika Verwendung finden.

Die neuen Carbacycline zeichnen sich außerdem noch durch Unterdrückung von Abstoßungsreaktionen und durch ihre antimetastatische Wirkung aus. Durch sie wird der Ductus Botalli (vor Operationen) offengehalten. Sie eignen sich ferner zur Durchfallbehandlung und zur Verbesserung des Stuhlganges.

Die Dosis des Verbindungen ist 1—1500 µg/kg/Tag, wenn sie am menschlichen Patienten verabreicht werden. Die Einheitsdosis für den pharmazeutischen akzeptablen Träger beträgt 0,01—100 mg.

Bei intravenöser Injektion an wachen, hypertonen Ratten in Dosen von 5, 20 und 100 µg/kg Körpergewicht zeigen die erfindungsgemäßen Verbindungen eine stärkere blutdrucksenkende und länger anhaltende Wirkung als $PGE_2$ und $PGA_2$, ohne wie $PGE_2$ Durchfälle oder $PGA_2$ kardiale Arrhythmien auszulösen.

Bei intravenöser Injektion an narkotisierten Kaninchen zeigen die erfindungsgemäßen Verbindungen in Vergleich zu $PGE_2$ und $PGA_2$ eine stärkere und erheblich länger anhaltende Blutdrucksenkung, ohne daß andere glattmuskuläre Organe oder Organfunktionen beeinflußt werden.

Für die parenterale Verabreichung werden sterile, injizierbare, wässrige oder ölige Lösungen benutzt. Für die orale Applikation sind beispielsweise Tabletten, Dragees oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen z.B. zur Herstellung von Blutdrucksenkern dienen.

Der Einheitsdosisbereich für die Ampulle ist 0,1—0,5 mg, für die Tablette 0,1—1 mg.

## Beispiel 1

5—(E)—{7-Hydroxy-6-[(E)-3-hydroxy-4-methyl-oct-1-en-6-inyl]-9-[6-amino-hexyl]-bicyclo[3.3.0]octan-3-yliden pentansäure

221 mg (0.5 mmol) 7α-Tetrahydropyran-2-yl-oxy)-6β-[(E)-4-methyl-3-(tetrahydropyran-2-yl-oxy)-oct-1-en-6-inyl]bicylo-[3.3.0]-oct-1-en-3-on und 12 mg $Cu(OAc)_2 \cdot H_2O$ in 6 ml abs. Tetrahydroduran wurden langsam bei −15° mit einem Grignard-Reagenz, bereitet aus 670 mg (2 mmol) 2,2,5,5-Tetramethyl-1-aza-2,5-disilacyclopentan-1-hexylbromid und 103 mg Magnesiumspänen in 2 ml abs. Äther, versetzt, bis über verschiedene Farbänderungen eine pechschwarze Farbe erreicht wurde.

Nach Aufarbeitung mit $NH_4Cl$ wurde das Rohprodukt entsprechend der folgenden Vorschrift für das Ausgangsmaterial nachsilyliert und das Rohprodukt, das nach D.C. noch 13% Ausgangsmaterial enthielt, eine Wittig-Reaktion mit 10 Äquivalenten 4-Carboxybutyltriphenylphosphoniumbromid/Kalium tert.-butylat in Dimethylsulfoxyd-Tetrahydrofuran = 2:1 unterworfen. Nach 3 Stunden Rühren bei 30° wurde mit Eiswasser versetzt, mit Citronensäure vorsichtig neutralisiert und mit Äther extrahiert. Nach Chromatographie an Silicagel erhielt man reines 5-E-Isomeres, das nach Abspaltung der Schutzgruppen mit $AcOH—H_2O—THF$ das gewünschte 5—E—Endprodukt ergab.

IR (Ölfilm) 3400—3000 $cm^{-1}$ ($NH_2$ und OH) 1710—1600 $cm^{-1}$ (Säurecarbonyl und Carboxylat)

2,2,5,5-Tetramethyl-aza-2,5-disilacyclopentan-1-hexylbromid wurde wie folgt dargestellt:

10 g (38,3 mmol) 6-Brom-1-hexylamin-hydrobromid und 8,25 g (38,3 mmol) 1,2-Bischlordimethylsilyläthan wurden in Gegenwart von 15,9 ml (115 mmol) Triäthylamin in 115 ml Methylenchlorid 3 Stunden bei 24° gerührt, wobei Triethylamin-hydrochlorid ausfiel. Nach Absaugen des Triäthylamin-hydrochlorids und Nachwaschen mit Methylenchlorid wurde abgedampft und der Rückstand in 50 ml trockenem Hexan

aufgenommen, wobei restliches Triäthylaminhydrochlorid ausfiel, das abfiltriert wurde. Nach Abdampfen wurden so 11,52 g (93,3%) erhalten, die bei 92—102°/0,05 mm destillierten. Das Destillat ist wie das Rohprodukt leicht trübe.

Beispiel 2

5-(E)-{7-(Tetrahydropyran-2-yl-oxy)-6-[(E)-4-methyl-3-(tetrahydropyran-2-yl-oxy)-oct-1-en-6-inyl]-9-[5-hydroxy-pentyl]-bicyclo-[3.3.0]-octan-3-yliden}-pentansäure

Zu 740 mg (1,67 mmol) 7α-Tetrahydropyran-2-yl-oxy)-6β-[(E)-4-methyl-3-(tetrahydropyran-2-yl-oxy)-oct-1-en-6-inyl]-bicylo[3.3.0]oct-1-en-3-on und 40,2 mg $Cu(OAc)_2 \cdot H_2O$ in 20 ml abs. Tetrahydrofuran wurde langsam bei −15° eine Grignard-Lösung, die aus 3,949 g 5-tert.-Butyldimethylislyloxypentylbromid und 689 mg Magnesiumspänen in 15 ml abs. Äther hergestellt worden war, innerhalb von 10 Minuten zugetropft, bis eine pechschwarze Färbung entstand. Nach Aufarbeitung wurde das Rohprodukt an einer Säule von ca. 150 g $SiO_2$ in hexan-Äther chromatographiert, wobei 932 mg = 86,4% reines bicyclisches Keton erhalten wurden. Diese 932 mg (1,445 mmol) setzte man analog Beispiel 1 mit über schüssigem 4-Carboxybutyltriphenylphosphoniumbromid um und trennte nach Aufarbeitung die isomeren Verbindungen durch Chromatographie in Hexan-Äther an 150 g feinem Silicagel, wobei reines (e)-Stereoisomeres erhatlen wurde. Durch Behandlung mit Tetrabutylammoniumfluoridlösung in THF verseifte die tert.-Butyl-dimethylsilyl-Schutzgruppe am C-9 des Bicyclooctangerüstes selektiv und es bildete sich die Titelverbindung. Die Umsetzung mit Essigsäure—$H_2O$—THF/80° führt dann zum freien Carbacyclinderivat 5(E){7-Hydroxy-6-[(E)-4-methyl-3-hydroxy-oct-1-en-6-inyl]-9[5-hydroxylpentyl]-bicyclo [3.3.0]-octan-3-yliden}-pentansäure.

IR (Ölfilm) 3300—3000 $cm^{-1}$ (OH).1740—1710 $cm^{-1}$ (Säure carbonyl/breit/).

5-tert-Butyldimethylsilyloxy-pentylbromid wurde wie folgt dargestellt:

10,45 g (50 mmol) 5-Bromvaleriansäureäthylester wurden bei 0° mit 1,043 g $LiAlH_4$ in 100 ml abs. Tetrahydrofuran reduziert, mit Eiswasser, 2 N $H_2SO_4$ aufgearbeitet und mit pentan, 20% Äther an einer Säule mit 275 g kieselgel chromatographiert, wobei 5,88 g 5-Brom-pentylalkohol erhalten wurden.

7,01 g (42 mmol) 5-Brom-pentylalkohol rührte man in 20 ml DMF mit 3,571 g Imidazol und 7,906 g (52,5 mmol) tert.-Butyl-dimethylsilylchlorid 2 Stunden bei 24°, setzte Eiswasser zu und extrahierte mit Hexan/Äther 1:1, wobei 12,58 g Rohprodukt erhalten wurden, das in Pentan nach Chromatographie an Silicagel 8,93 g (75%) der Titelverbindung ergab.

**Patentansprüche**

1. Carbacyclinderivate der allgemeinen Formel I

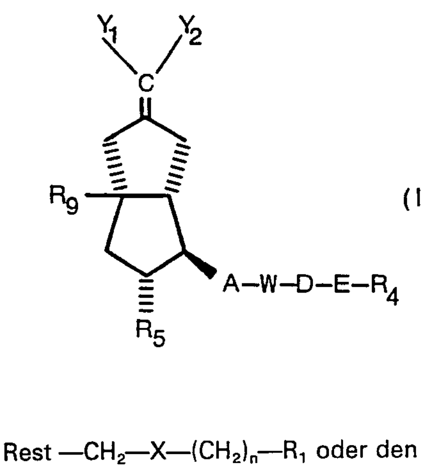

(I),

worin

$Y_1$ den Rest $—CH_2—X—(CH_2)_n—R_1$ oder den Rest 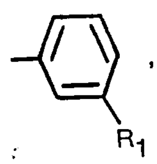 ,

n 1 oder 3,

$R_1$ den Rest 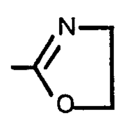 , den Rest 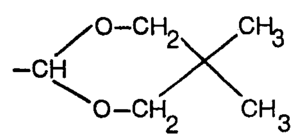 ,

$—COCH_3$, $COOR_2$, wobei $R_2$ Wasserstoff oder gegebenenfalls durch Halogen, Phenyl, $C_1—C_4$—Alkoxy oder $C_1—C_4$—Dialkyl-amino substituiertes Alkyl mit 1—10 C-Atomen, Cycloalkyl, Aryl oder einen heterocyclischen Rest bedeuten kann, oder den Rest $CONHR_3$ mit $R_3$ in der Bedeutung Wasserstoff oder eines Alkanoyl- oder Alkansulfonylrestes mit je 1—10 C-Atomen darstellt,

$R_9$ den Rest $—(CH_2)_m—R_6$ oder den Rest

$$—(CH_2)_{m-o}—[Z_1—(CH_2)_{m-p}]_x—[Z_2—(CH_2)_{m-q}]_y—R_6,$$

m = 2—20

o, p und q ≤ 19

x, y = 0, 1 oder 2,

$Z_1$ eine cis—CH=CH-Gruppe, eine trans—CH=CH—Gruppe oder eine —C≡C—Gruppe darstellt, wobei jede dieser Gruppen mindestens durch eine Methylengruppe vom C-9 Kohlenstoffatom des Carbacyclinbicyclus getrennt sein muß,

$Z_2$ Sauerstoff, Schwefel, eine NH— oder eine N-Methylgruppe bedeutet,

$R_6$ Amino, Methylamino, Hydroxy, Carboxy oder Merkapto bedeutet,

X eine Sauerstoffatom oder eine Methylengruppe,

$Y_2$ Wasserstoff oder Fluor,

A eine $—CH_2—CH_2—$, trans —CH=CH— oder —C≡C—Gruppe,

W eine freie oder funktionell abgewandelte Hydroxymethylengruppe oder eine freie oder funktionell abgewandelte

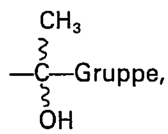

wobei die OH-Gruppe α- oder β-ständig sein kann,

D die Gruppe

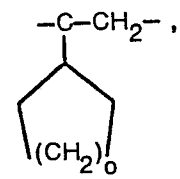

eine geradkettige, gesättigte Alkylengruppe mit 1—5 C-Atomen, eine verzweigte gesättigte oder eine geradkettige oder verzweigte ungsättigte Alkylengruppe mit 2—5 C-Atomen, die gegebenenfalls durch Fluoratome substituiert sein können,

o 1, 2 oder 3 bedeutet,

E eine Direktbindung, eine —C≡C—Gruppe oder eine $—CH=CR_7—$ Gruppe darstellt, wobei $R_7$ ein Wasserstoffatom, eine Alkylgruppe mit 1—5 C-Atomen oder Halogen bedeuten,

$R_4$ eine Alkylgruppe mit 1—10 C-Atomen, eine Cycloalkylgruppe mit 3—10 C-Atomen oder eine gegebenenfalls substituierte Arylgruppe mit 6—10 C-Atomen oder eine heterocyclische Gruppe,

$R_5$ eine freie oder funktionell abgewandelte Hydroxygruppe, und, falls $R_2$ die Bedeutung eines Wasserstoffatoms hat, deren Salze mit physiologisch verträglichen Basen bedeuten.

2. Verfahren zur Herstellung von Carbacyclinderivaten, der allgemeinen Formel I, dadurch gekennzeichnet, daß man in an sich bekannter Weise a) eine Verbindung der allgemeinen Formel IV

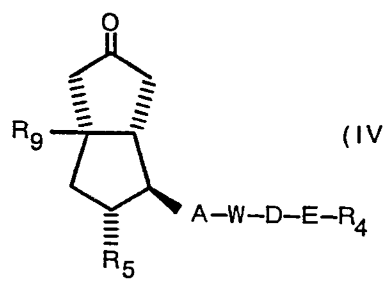

(IV),

worin A, W, D, E, $R_4$, $R_5$ und $R_9$ die bereits angegebenen Bedeutungen haben, und mit einem Wittigreagenz der allgemeinen Formeln V oder VI oder einem Dianion der Formel VII

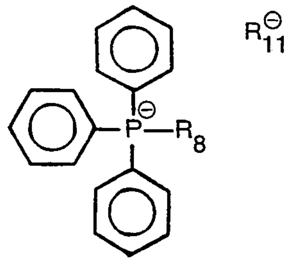

(V),

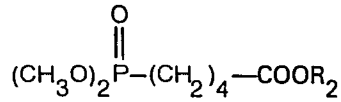

(VI)

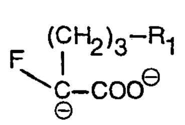

(VII)

worin

$R_2$ die genannte Bedeutung und $R_8$ die Reste —$CH_2$—$CH_2$—X—

$(CH_2)_n-R_1$ oder 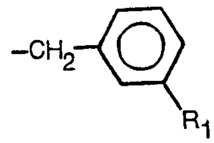

mit den bereits erwähnten Bedeutungen für X, n und $R_1$ und $R_{11}$ Brom oder Chlor bedeuten, in Gegenwart von K-tert.-Butylat umsetzt oder b) eine Verbindung der allgemeinen Formel VIII, die man aus dem entsprechenden 4-Ester durch DIBAH-Reduktion erhält.

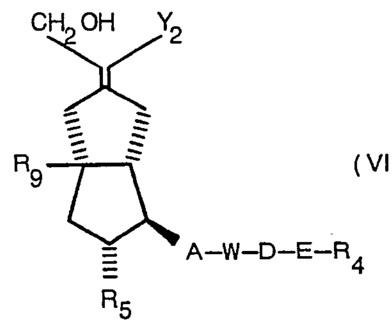

(VIII),

worin A, W, D, E, $R_4$, $R_5$, $R_9$ und $Y_2$ die bereits angegebenen Bedeutungen haben, gegebenenfalls nach Schutz anwesender freier Hydroxy- oder Aminogruppen mit einem Halogenalkansäurederivat der allgemeinen Formel IX

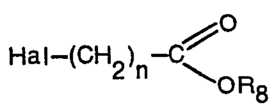

(IX),

wobei n 1 oder 3, Hal ein Chlor- oder Bromatom und $R_8$ einen Alkylrest mit 1—4 C-Atomen oder ein Alkalimetall bedeuten, in Gegenwart einer Base veräthert und gegebenenfalls anschließend in beliebiger Reihenfolge Isomere trennt und/oder geschützte Hydroxygruppen freisetzt und/oder freie Hydroxygruppen verestert, veräthert und/oder eine freie Carboxylgruppe verestert und/oder eine veresterte Carboxylgruppe verseift oder eine Carboxylgruppe in ein Amid oder mit einer physiologisch verträglichen Base in ein Salz überführt.

3. Arzneimittel, enthaltend ein oder mehrere Verbindungen gemäß Anspruch 1 und übliche Hilfs- und Trägerstoffe.

4. 5—(E)—{7-Hydroxy-6-[(E)-3-hydroxy-4-methyl-oct-1-en-6-inyl]-9-[6-amino-hexyl]-bicyclo[3.3.0]octan-3-yliden}-pentansäure.

5. 5-(E)-{7-(Tetrahydropyran-2-yl-oxy)-6-[(E)-4-methyl-3-(tetrahyhdropyran-2-yl-oxy)-oct-1-en-6-inyl]-9-[5-hydroxy-pentyl]-bicyclo-[3.3.0]-octan-3-yliden}-pentansäure.

## Revendications

1. Carbacyclines répondant à la formule générale I:

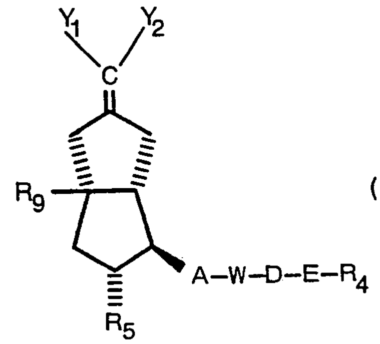

(I),

dans laquelle:

$Y_1$ représente un radical $-CH_2-X-(CH_2)_n-R_1$ ou 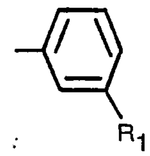 ,

n est égal à 1 ou à 3,

$R_1$ représente un radical 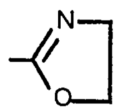 , 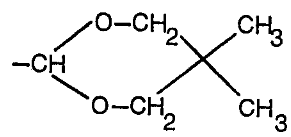 ,

$-COCH_3$, $-COOR_2$ (dans ce dernier $R_2$ représente l'hydrogène, un alkyle en $C_1-C_{10}$ éventuellement porteur d'un halogène, d'un phényle, d'un alcoxy en $C_1-C_4$ ou d'un di-$C_1-C_4$-alkylamino, un cycloalkyle, un aryle ou un radical hétérocyclique) ou $-CONHR_3$ (dans ce dernier $R_3$ représente l'hydrogène ou un radical alcanoyle ou alcane-sulfonyle contenant de 1 à 10 atomes de carbone),

$R_9$ représente un radical $-(CH_2)_m-R_6$ ou un radical:

$$-(CH_2)_{m-o}-[Z_1-(CH_2)_{m-p}]_x-[Z_2-(CH_2)_{m-q}]_y-R_6,$$

m désigne un nombre de 2 à 20,

o, p et désignent des nombres au plus égaux à 19,

x et y sont égaux chacun à 0, à 1 ou à 2,

$Z_1$ représente un radical $-CH=CH-$ cis, un radical $-CH=CH-$trans ou un radical $-C\equiv C-$, chacun de ces radicaux devant être séparés de l'atome de carbone C-9 du système bicyclique de la carbacycline par au moins un radical méthylène,

$Z_2$ représente l'oxygène, le soufre, un radical $-NH-$ ou un radical $-N(\text{méthyl})-$,

$R_6$ représente un radical amino, méthylamino, hydroxy, carboxy ou mercapto,

X représente un atome d'oxygène ou un radical méthylène,

$Y_2$ représente l'hydrogène ou le fluor,

A représente un radical $-CH_2-CH_2-$, un radical $-CH=CH-$ trans ou un radical $-C\equiv C-$,

W représente un radical hydroxyméthylène libre ou sous la forme d'un dérivé fonctionnel, ou un radical:

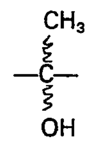

libre ou sous la forme d'un dérivé fonctionnel, le radical $-OH$ pouvant avoir la configuration α ou la configuration β,

D représente un radical

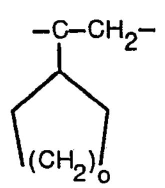 ,

ou un radical alkylène saturé linéaire en $C_1-C_5$ ou un radical alkylène en $C_2-C_5$ saturé ramifié ou insaturé linéaire ou ramifié, chacun de ces radicaux portant éventuellement des atomes de fluor,

o désigne un nombre égale à 1, à 2 ou à 3,

E représente une liaison directe, un radical $-C\equiv C-$ ou un radical $-CH=CR_7-$ dans lequel $R_7$ représente un atome d'hydrogène, un alkyle en $C_1-C_5$ ou un halogène,

$R_4$ représente un alkyle contenant de 1 à 10 atomes de carbone, un cycloalkyle contenant de 3 à 10 atomes de carbone, un aryle en $C_6-C_{10}$ éventuellement substitué ou un radical hétérocyclique, et

$R_5$ représente un radical hydroxy libre ou sous la forme d'un dérivé fonctionnel,

et, dasns la cas où $R_2$ désigne un atome d'hydrogène, les sels qu'elles forment avec des bases acceptables du point de vue physiologique.

2. Procédé pour préparer des carbacyclines de formule générale I, procédé caractérisé en ce que, en opérant de manière connue:

a) on fait réagir un composé répondant à la formule générale IV:

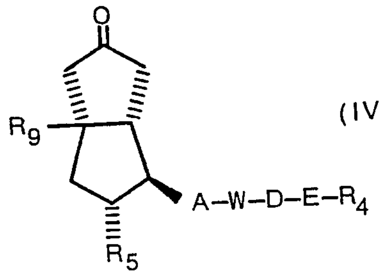

(IV),

dans laquelle A, W, D, E, $R_4$, $R_5$ et $R_9$ ont les significations qui leur ont été données ci-dessus, avec un réactif de Wittig de formule générale V ou VI ou avec un dianion de formule VII:

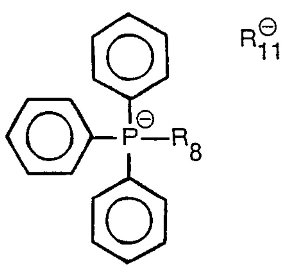

(V),

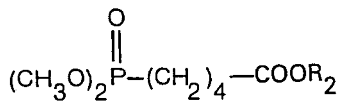

(VI)

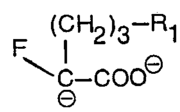

(VII)

formules dans lesquelles $R_2$ a la signification indiquée ci-dessus, $R_8$ représente un radical

$-CH_2-CH_2-X-(CH_2)_n-R_1$ ou un radical 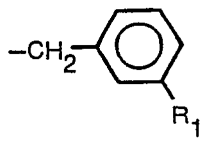 ,

X, n et $R_1$ ont les significations qui leur ont été données ci-dessus, et $R_{11}$ représente le brome ou le chlore, en présence de tert-butylate de potassium, ou

b) on éthérifie, en présence d'une base, un composé répondant à la formule générale VIII:

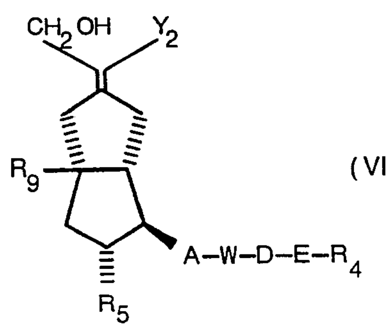

(VIII),

dans laquelle A, W, D, E, $R_4$, $R_5$, $R_9$ et $Y_2$ ont les significations qui leur ont déjà été données, (composé obtenu à partir de l'ester en 4 correspondant, par réduction au moyen du DIBAH), éventuellement après avoir protégé d'éventuels radicaux hydroxy ou amino libres, avec un dérivé d'acide halogéno-alcanoïque répondant à la formule générale IX:

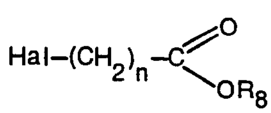

(IX),

dans laquelle n est égal à 1 ou à 3, Hal représente un atome de chlore ou de brome et $R_8$ représente un radical alkyle contenant de 1 à 4 atomes de carbone ou un atome de métal alcalin, puis, éventuellement, en opérant dans l'ordre que l'on veut, on sépare des isomères et/ou on libère des radicaux hydroxy protégés et/ou on estérifie ou éthérifie des radicaux hydroxy libres et/ou on estérifie un radical carboxy libre et/ou on saponifie un radical carboxy estérifié ou on transforme un radical carboxy en un amide ou, au moyen d'une base acceptable du point de vue physiologique, en un sel.

3. Médicament contenant un ou plusieurs composés selon la revendication 1 et des adjuvants et excipients usuels.

4. Acide {hydroxy-7 [hydroxy-3 méthyl-4 octène-1 yne-6 yl-(E)]-6 (amino-6 hexyl)-1 bicyclo[3.3.0] octylidène-3}-5 pentanoïque (E).

5. Acide {(tétrahydropyrannyl-2 oxy-7 [méthyl-4 (tétrahydropyrannyl-2 oxy)-3 octène-1 yne-6 yl-(E)]-6 (hydroxy-5 pentyl)-1 bicyclo[3.3.0]octylidène-3}-5 pentanoïque.

## Claims

1. Carbacyclin derivatives of the general formula I

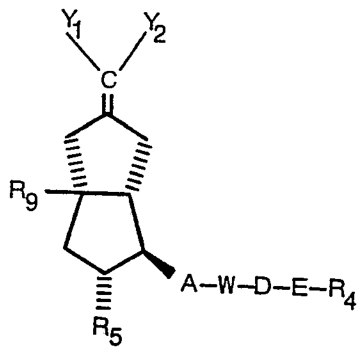 (I),

in which

$Y_1$ represents the radical $-CH_2-X-(CH_2)_n-R_1$ or the radical 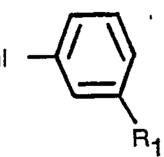,

n is 1 or 3

$R_1$ represents the radical 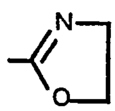, the radical 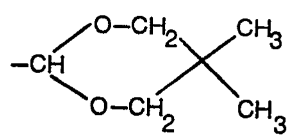,

$-COCH_3$, $COOR_2$ wherein $R_2$ may represent hydrogen, alkyl having from 1 to 10 C-atoms optionally substituted by halogen, phenyl, $C_1-C_4$-alkoxy or by $C_1-C_4$-dialkylamino, cycloalkyl, aryl or a heterocyclic radical, or $R_1$ represents the radical $CONHR_3$ wherein $R_3$ represents hydrogen or an alkanoyl or alkanesulfonyl radical each having from 1 to 10 C-atoms,

$R_9$ represents the radical $-(CH_2)_m-R_6$ or the radical

$$-(CH_2)_{m-o}-[Z_1-(CH_2)_{m-p}]_x-[Z_2-(CH_2)_{m-q}]_y-R_6,$$

m = 2 to 20 and

0, p and q $\leq$ 19,

x and y = 0, 1 or 2,

$Z_1$ represents a cis$-CH=CH-$ group, a trans $-CH=CH-$ group or a $-C\equiv C-$ group, each of which must be separated from the C-9 carbon atom of the carbacyclin nucleus by at least a methylene group,

$Z_2$ represents oxygen, sulphur, an NH- or an N-methyl- group,

$R_6$ represents amino, methylamino, hydroxy, carboxy or mercapto,

X represents an oxygen atom or a methylene group,

$Y_2$ represents hydrogen or fluorine

A represents a $-CH_2-CH_2-$, trans$-CH=CH-$ or $-C\equiv C-$ group

W represents a free or functionally modified hydroxy-methylene group or a free or functionally modified

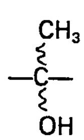

group wherein the OH group may be in the α- or β-configuration,

D represents the group

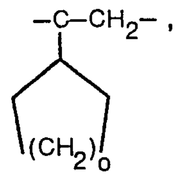

a straight-chain saturated alkylene group having from 1 to 5 C-atoms, a branched saturated, or a straight-chain or branched unsaturated, alkylene group having from 2 to 5 C-atoms, each of which may optionally be substituted by fluorine atoms,

O is 1, 2 or 3,

E is a direct bond, a —C≡C— group or a —CH=CR$_7$— group wherein $R_7$ represents a hydrogen atom, an alkyl group having from 1 to 5 C-atoms, or halogen,

$R_4$ represents an alkyl group having from 1 to 10 C-atoms, a cycloalkyl group having from 3 to 10 C-Atoms or an optionally substituted aryl group having from 6 to 10 C-atoms, or a heterocyclic group, and

$R_5$ represents a free or functionally modified hydroxy group,

and when $R_2$ is a hydrogen atom, salts thereof with physiologically tolerable bases.

2. Process for the preparation of carbacyclin derivatives of the general formula I, characterised in that, in a manner known *per se*, a) a compound of the general formula IV

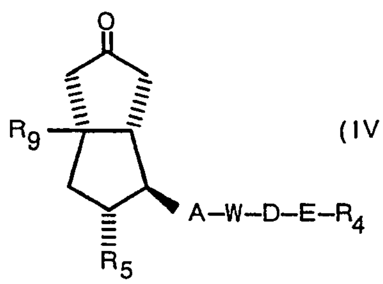 (IV),

in which A, W, D, E, $R_4$, $R_5$ and $R_9$ have the meanings given hereinbefore, is reacted with a Wittig reagent of the general formula V or VI or a dianion of formula VII

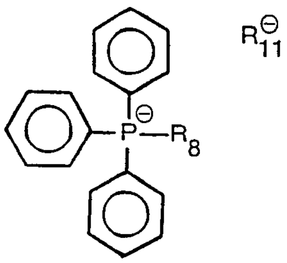 (V),

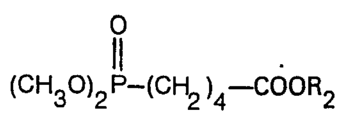 (VI)

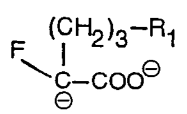 (VII)

wherein

$R_2$ has the meaning given and $R_8$ represents the radical

$-CH_2-CH_2-X-(CH_2)_n-R_1$ or 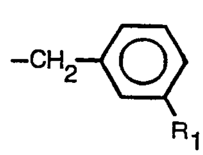

in which X, n and $R_1$ have the meanings given hereinbefore and $R_{11}$ is bromine or chlorine, in the presence of K-tert.-butoxide,

or

b) a compound of the general formula VIII, which is obtained from the corresponding 4-ester by DIBAH reduction,

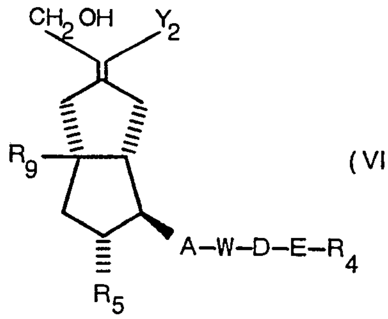

(VIII),

wherein A, W, D, E, $R_4$, $R_5$, $R_9$ and $Y_2$ have the meanings given hereinbefore, optionally after protecting any free hydroxy or amino groups present, is etherified in the presence of a base with a haloalkanoic acid derivative of the general formula IX

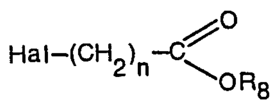

(IX),

wherein n is 1 or 3, Hal represents a chlorine or bromine atom and $R_8$ represents an alkyl radical having from 1 to 4 C-atoms, or an alkali metal, and optionally, subsequently, in any order, isomers are separated and/or protected hydroxy groups are freed and/or free hydroxy groups are esterified or etherified and/or a free carboxy group is esterified and/or an esterified carboxy group is hydrolysed or a carboxy group is converted into an amide or converted by means of a physiologically tolerable base into a salt.

3. Medicaments containing one or more compounds according to claim 1 and conventional excipients and carriers.

4. 5-(E)-{7-Hydroxy-6-[(E)-3-hydroxy-4-methyloct-1-en-6-ynyl]-9-[6-aminohexyl]-bicyclo[3.3.0]octan-3-ylidene} pentanoic acid.

5. 5-(E)-{7-(Tetrahydropyran-2-yloxy)-6-[(E)-4-methyl-3-(tetrahydropyran-2-yloxy)-oct-1-en-6-ynyl]-9-[5-hydroxypentyl]-bicyclo[3.3.0]octan-3-ylidene} pentanoic acid.